# EUROPEAN PATENT SPECIFICATION

(11) **EP 3 385 710 B1**
(45) Date of publication and mention of the grant of the patent: **01.12.2021**
(21) Application number: 17164892.6
(22) Date of filing: 05.04.2017
(51) Int. Cl.: G01N 33/00, F24F 11/32, F24F 11/38, F24F 11/00, A61L 9/00, F24F 110/50, F24F 110/66

(54) **CONTAMINATION PREVENTION IN A BUILDING**
VORBEUGUNG VON KONTAMINATION IN EINEM GEBÄUDE
PRÉVENTION DE CONTAMINATION DANS UN BÂTIMENT

(43) Date of publication of application: 10.10.2018
(73) Proprietor: InsightAir Europe, 1680 Sofia (BG)
(72) Inventor: Mertens, Kristin, 2250 Olen (BE)
(74) Representative: IP HILLS NV

(56) References cited:
- EP-A1- 1 574 836
- US-A- 5 597 354
- US-A1- 2011 064 605
- US-A1- 2013 171 687
- US-A1- 2013 323 781

## Description

### Field of the Invention

The present invention generally relates to the prevention of contamination in a building by micro-organisms such as fungi and bacteria. More particular, the invention relates to a method and building ventilation system for preventing such contamination.

### Background of the Invention

Such micro-organisms may for example emerge from patients in hospitals and rapidly contaminate other patients. Such harmful micro-organisms may also emerge from decaying food products and spread again rapidly throughout a building.

Fast detection and identification of harmful micro-organisms is therefore important such that further spreading can be prevented. One way of detection is by a microbiological culture wherein a volume of air is guided over a microbiological medium and the micro-organisms are further incubated under controlled conditions for a certain incubation time. Thereafter, the contamination can be identified.

A problem with such microbiological cultures is that they take a long time to perform.

Micro-organisms may also be detected indirectly by Volatile Organic Component, VOC, sensors. VOCs are produced by micro-organisms and the presence of VOCs may thus indicate the presence of micro-organisms and thus microbiological contamination.

In US2015/0156031 a smart hazard detector is disclosed that comprises air quality sensors that "sniff" for VOCs. Several of these detectors are then placed inside and outside a building. The detectors communicate the sensor information to a central server. The central server then establishes whether the air outside is purer than the air inside. If so, the central server instructs a smart thermostat to open a vent to permit fresh air into the house, otherwise it instructs the thermostat to recirculate air in the home and to not draw in outside air.

A problem of the above solution is that VOC sensors are not precise, i.e., a single sensor cannot differentiate all different VOCs. Furthermore, not all VOCs are an indication of microbiological contamination. The above solution may thus lead to false positives. In a hospital or professional kitchen environment, such false positives are highly undesirable. A further disadvantage of the above solution is that the forced air recirculation may spread a microbiological contamination to other sub-parts of a building.

US2011064605A1 discloses methods of sanitizing structures, buildings, passenger occupiable vehicles, and other enclosed or enclosable spaces. More particularly, the disclosure relates to a method for killing and/or removing pests and their allergens, bacteria, viruses, fungi, molds, volatile organic compounds and other dangerous substances, from such objects or enclosures.

US2013323781A1 discloses a device for detecting a fungal contamination in an internal environment.

### Summary of the Invention

It is an object of the present invention to overcome the above shortcomings and to provide a solution that prevents microbiological contamination in a building in a fast and accurate way.

According to a first aspect, the invention relates to the method according to claim 1.

A two-stage approach is thus proposed comprising a two-stage detection. In a first stage, a faster but less accurate VOC detection mechanism is used by the use of a VOC sensor. By the VOC sensor, a microbiological contamination is detected in a sub-part of a building, e.g., a room, which is ventilated by the building's ventilation system. The VOC sensor may be positioned inside the room or within the exit part of the ventilation system. This way, the VOC sensor measures the contamination on a volume of air supplied by the ventilation system. When a contamination by the VOC sensor is detected, a first preventive action is taken. This preventive action is a first measure to prevent further contamination. The first action may be related to the prevention of people entering the first sub-part or to the prevention of ventilated air from the sub-part to contaminate further parts of the building by the ventilation system. At the same time, a second measurement is triggered by the first measurement. The second measurement is more precise as it uses a larger volume of air to perform the measurement. When this second measurement is also positive, a second, more extensive, action is taken to prevent the further contamination. When the second measurement is negative, the first action is undone such that the initial situation of before the detection is restored.

An advantage of the proposed method is that a short detection time is achieved by the use of the VOC sensor while achieving a very precise detection that is not realizable by the VOC sensor. Furthermore, unnecessary and unneeded annoyance by the second more elaborate action is avoided in the case of false positive by the VOC sensor. All this leads to an accurate prevention mechanism while minimizing the burden caused by false positives.

A further advantage is that an integrated ventilation solution is obtained, combining building ventilation with the prevention of microbiological contamination.

While a VOC sensor can only base its measurement on a small volume of air passing by the sensor, all the air from the first sub-part will pass by the air filter of the ventilation system. Furthermore, the air filter will further accumulate the micro-biological contamination over time. It is therefore an advantage that a more precise measurement of the contamination can be performed by the use of the air filter as a first culture medium. It is further advantage that the method can be easily applied to existing ventilation systems as they typically comprise one or more of these air filters.

When the air from the first sub-part does not pass an air filter, the air may be channelled through an air filter by changing the air exit channel upon the detection of the contamination by the VOC sensor.

The advantage of this is that a culture medium may be used that is specifically designed for the purpose of performing a microbiological culture on it thereby increasing the accuracy of the second measurement.

The second action may then comprise removing a source that causes the contamination from the first ventilated sub-part. If the second measurement is negative, the first action can be undone.

According to an embodiment, the taking the first action comprises adapting the ventilation system such that the sub-part is no longer ventilated thereby preventing contamination of other sub-parts of the building through the ventilation system.

This may for example be done by closing a ventilation outlet in the respective sub-part either manually or automatically. As the room is no longer ventilated, contaminated air will not be spread further into the building. This has the advantage that a further possible contamination is prevented without affecting other rooms.

Additionally, taking the first action may further comprise adapting the ventilation such that other sub-parts that are in contact with the first ventilated sub-part are no longer ventilated.

In other words, when air from the ventilated sub-parts travels through other sub-parts of the building, the ventilation of those rooms is also stopped. This way, a potential spreading of the contamination is further prevented without affecting more sub-parts than necessary.

According to an embodiment, the first ventilated sub-part is a patient room and taking the first action comprises preventing the patient from leaving the room.

The presence of certain VOCs in the air of a patient room may be an indication of a disease or infection by micro-organisms. If the disease or infection is contagious, the patient is considered as a possible source of contamination and prevented from leaving the room as the first action. This way, further contamination is prevented without much discomfort for the patient.

According to an alternative embodiment, the first ventilated sub-part comprises food products and taking the first action comprises marking the food products as possibly contaminated.

This situation will generally occur in a room where food products are prepared or stored such as in a kitchen. Again, the presence of certain VOCs may be an indication of decay in the food products, for example by fungi or bacteria. By marking the food, further contamination by displacing the food products to other locations is prevented. When ventilation is further stopped as the further first action, further contamination through the air is also prevented.

The method according to any one for the preceding claims wherein the taking a first action further comprises marking other sub-parts of the building through which air from the first ventilated sub-part flows as possibly contaminated.

In other word, not only the first sub-part of the building is marked, but also other parts that may be contaminated by ventilation air from the first sub-part. By marking this parts, people are warned of possible contamination and will act more cautious. Again, this further prevents a possible contamination upon the detection by the VOC sensor in a non-obtrusive way.

### Brief Description of the Drawings

Fig. 1 illustrates a building comprising a ventilation system according to an embodiment of the invention; and
Fig. 2 illustrates the building of Fig. 1 after steps according to an embodiment of the invention have been performed; and
Fig. 3 illustrates steps performed according to an embodiment of the invention for preventing contamination of a building; and
Fig. 4 illustrates a suitable computing system for performing steps according to embodiments of the invention.

### Detailed Description of Embodiment(s)

The present invention relates to the detection of a microbiological contamination in a building and the prevention of the further spreading of the contamination throughout the building. A contamination may for example be a direct contamination of a living organism or an organic product by for example an infection, bacteria or fungi. Such contamination will result in the release of VOCs into the air, either directly or indirectly.

If air is contaminated by micro-organisms in a certain sub-part of a building, then other parts of the building may be contaminated by the transportation of the contaminated air throughout the building, for example by a ventilation system. If a living organism such as a human or animal is contaminated, the contamination may be further spread by direct or indirect contact. If other organic material such as food products is contaminated, the contamination may be spread by processing the food products and consumption of the processed food. According to embodiment of the invention, all these types of contaminations may be detected, prevented from further spreading and removed such that the contamination no longer exists.

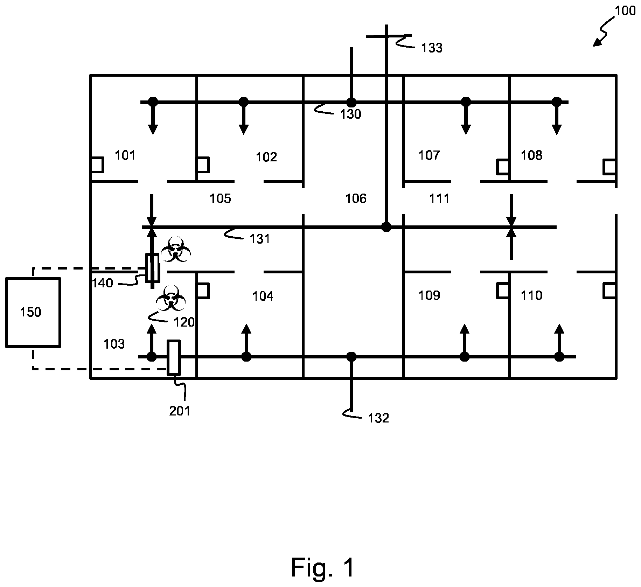

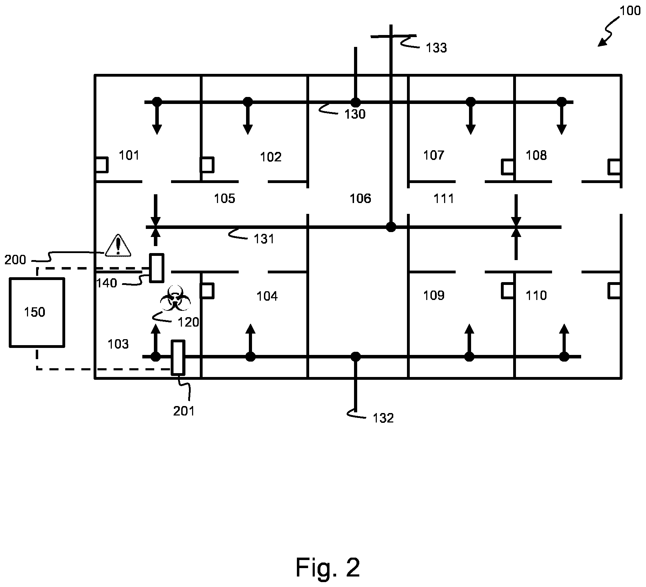

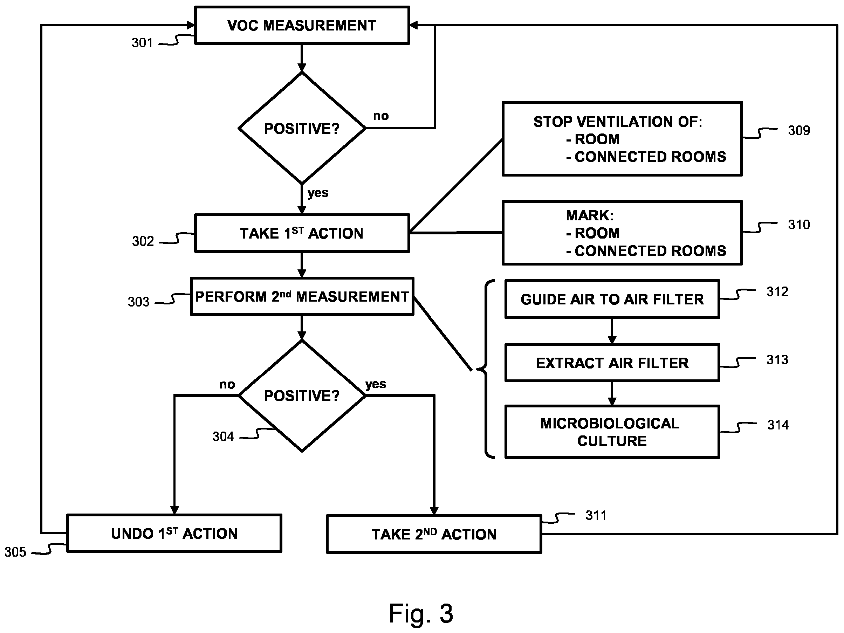

An embodiment according to the present invention will now be explained by reference to Fig. 1, 2 and 3. Both Fig. 1 and Fig. 2 illustrate the same building 100 comprising a ventilation system. In Fig. 1 a situation in the building is depicted wherein a contamination 120 is present in the building. In Fig. 2, a situation is depicted where certain measures are taken by performing steps according to an embodiment of the invention. These step are illustrated in the flow diagram of Fig. 3.

Fig. 1 and Fig. 2 illustrate a general layout of a building 100. The building comprises several rooms 101-104 and 107-110 and corridors 105, 106 and 111 which are all considered as sub-parts of the building. All sub-parts are ventilated by a ventilation system. Main ventilation inlet 132 supplies fresh air into rooms 103, 104 and 109, 110. Main ventilation inlet 130 supplies fresh air into rooms 101, 102, 107 and 108. Corridors 105 and 111 each comprise a ventilation outlet which extracts air from the corridors. By the ventilation system fresh air thus flows from the rooms into the corridors where it is extracted. The ventilation system may further comprise air filters for filtering the air, either at the intake or outtake part. In building 100, an air filter 133 is incorporated in the outtake part 131 for filtering the outgoing air.

Rooms 101-104 and 107-110 further comprise a VOC sensor 140, i.e., a sensor for sensing the amount of volatile organic compounds in the air. Different types of such sensors exist depending on the type of VOC that needs to be measured or the required accuracy of the measurement. The VOC sensor is selected depending on the type of contamination that is to be measured. The sensor may be selected to detect biologically generated VOCs in order to further detect possible contamination from micro-organisms such as fungi or bacteria. A VOC sensor provides an almost instant detection of VOCs, i.e., no further analysis in a laboratory or integration or incubation time is needed. In Fig. 1 and 2, only VOC sensor 140 is referenced, but the other rooms 101-104 and 107-110 may also comprise such a VOC sensor. The VOC sensor 140 may be placed separate in the room, for example on a wall or the ceiling or the VOC sensor 140 may be integrated into the ventilation system, e.g. locating in the air inlet 132 or air outlet 131. VOC sensor 140 is placed such that at least a volume of air entering the room 103 from the air inlet passes the VOC sensor 140. This way, the ventilation system forces air to pass by the VOC sensors such that a new contamination can be detected rapidly.

Sub-parts of the building 100 may further be cut off from the ventilation system, for example by manual or automated closing of the air inlets and/or outlets. In Fig. 1 a valve 201 is illustrated in the inlet part 132 ventilating the room 103. Such valves may be placed in all sub-parts separately in order to accurately control the ventilation of each sub-part or may be placed such that larger portions of the building 100 can be cut-off from the ventilation system.

The ventilation system may further be controlled by a controller 150 that is configured to operate and monitor parts of the ventilation system such as for example read out the VOC sensors 140, open or close the valve 201, read the status of a filter 133 and control the amount of air from the air inlets.

Fig. 3 shows steps performed for preventing contamination in a building comprising a ventilation system according to an embodiment of the invention. The steps will be further illustrated by reference to the building 100 and the ventilation system of Fig. 1 and Fig. 2. In the building 100, during step 301, the amount of VOCs are measured, for example constantly or periodically. At a certain moment, VOC sensor 140 measures a contamination 120 present in room 103 based on a volume of air distributed through the room by the ventilation system. In this case, the VOC sensor 140 is positioned at the air outlet and will thus detect contamination 120. When the contamination is positive, the method proceeds to step 302 wherein a first preventive action is taken in order to prevent spreading of the contamination.

A first type of action may be to stop 309 the ventilation system in order to prevent further contamination in the building 100. This may be done by closing valve 201 such that ventilation in room 103 is stopped and contaminated air cannot be spread into corridor 131 by the ventilation system. Alternative, the ventilation in larger parts of the building may be stopped. For example, ventilation inlet 132 and outlet 131 may be completely closed.

A second type of action may be to mark places in the building 100 based on the detection by the VOC sensor 140. For example, when room 103 is a patient room in a hospital, the entrance of the room may be marked by a warning sign 200 such that people outside room 103 are warned about the possible contamination. In another example, the room 103 may be a place where food is processed or stored such as a kitchen. In this case, the food stored inside room 103 may be marked by a warning sign 200 such that the food is no longer used or removed from the room 103. Warning signs 200 may further be applicable to multiple rooms or sub-parts of the building 100. For example, a sign 200 may be placed at the entrance of corridor 105. Warning signs 200 may be electronically operated warning signs such as a red light positioned above the entrance of rooms or corridors. The second type of action may further be combined with the first type of action as a combined preventive action.

The positive VOC measurement further triggers the next step 303 wherein a further second measurement of the contamination is performed. The second measurement is a more precise measurement of the contamination, but also takes a longer time than the measurement by the VOC sensor. Such a more precise measurement is obtained by taking a larger volume of air into account then in the first VOC measurement under step 301. According to a further embodiment, the second measurement may be done according to steps 312 to 314. In step 312, the larger volume of air is guided through an air filter serving as a integration or culture medium. Such an air filter 313 may be positioned in the outlet part of the ventilation system. As almost all air from the contaminated room 103 will pass through the air filter, a much larger volume of air will pass through the air filter 313 then would pass by the VOC sensor 140. After the VOC sensor 140 has detected the contamination, the air filter is extracted from the ventilation system. This extraction may be done directly in response to the first detection or after a certain time period such that more contamination can be accumulated into the air filter. The air filter is then used in step 314 to perform a microbiological culture as second measurement. Air filter 313 may further be inserted into the ventilation system based on the first detection by the VOC sensor 140. In this case, the air filter 313 only serves for the purpose of the second detection and not for the filtering of air. In such a case, an air filter specifically suited for the accumulation of certain types of contamination may be used thereby obtaining a more precise second measurement.

When the outcome of the second measurement is negative 304, then the first VOC measurement was a false positive and the first action of step 302 is undone in step 305. When rooms or material have been marked by a sign 200, the signs are removed. When the ventilation of one or more rooms was stopped, then the ventilation in these rooms is restored.

When the outcome of the second measurement is positive 304, then a further second action is taken under step 311. Such second action may comprise the removal of the source of the contamination. For example, when the contamination was by a food product, the food product is removed from the respective room and destroyed or disposed. Or for example, when the contamination was by a patient, the patient may be further examined and/or put in isolation in order to prevent further contamination.

Several of the steps according to the above embodiments may be performed automatically by the controller 150 such as for example the VOC measurement 301, the 1^{st} action 302, the 2^{nd} measurement 303 and the undoing of the first action 305.

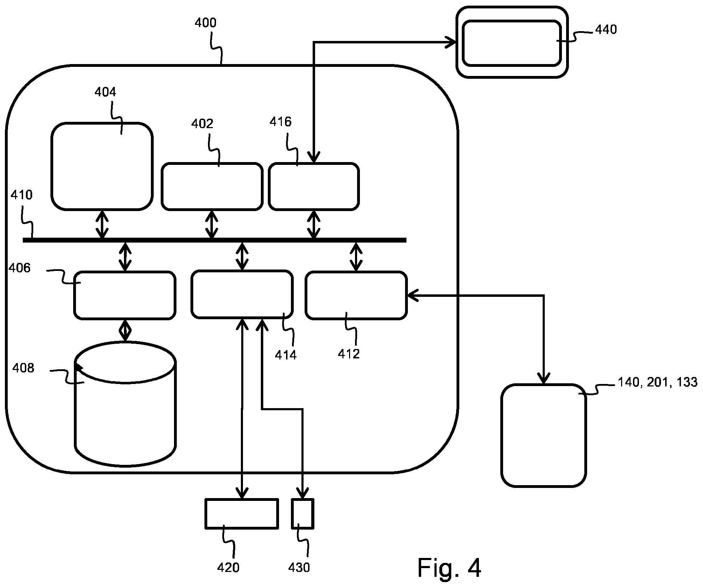

Fig. 4 shows a computing system 400 according to an embodiment of the invention. Computing system 400 is suitable for performing the steps by the controller 150 and thus for performing the steps according to the above embodiments. Computing system 400 may in general be formed as a suitable general purpose computer and comprise a bus 410, a processor 402, a local memory 404, one or more optional input interfaces 414, one or more optional output interfaces 416, a communication interface 412, a storage element interface 406 and one or more storage elements 408. Bus 410 may comprise one or more conductors that permit communication among the components of the computing system 400. Processor 402 may include any type of conventional processor or microprocessor that interprets and executes programming instructions. Local memory 404 may include a random access memory (RAM) or another type of dynamic storage device that stores information and instructions for execution by processor 402 and/or a read only memory (ROM) or another type of static storage device that stores static information and instructions for use by processor 402. Input interface 414 may comprise one or more conventional mechanisms that permit an operator to input information to the computing device 400, such as a keyboard 420, a mouse 430, a pen, voice recognition and/or biometric mechanisms, etc. Output interface 416 may comprise one or more conventional mechanisms that output information to the operator, such as a display 440, etc. Communication interface 412 may comprise any transceiver-like mechanism such as for example one or more Ethernet interfaces that enables computing system 400 to communicate with other devices and/or systems, for example with VOC sensor 140, valve 201 or filter 133. The communication interface 412 of computing system 400 may be connected to such another computing system by means of a local area network (LAN) or a wide area network (WAN) such as for example the internet. Storage element interface 406 may comprise a storage interface such as for example a Serial Advanced Technology Attachment (SATA) interface or a Small Computer System Interface (SCSI) for connecting bus 410 to one or more storage elements 408, such as one or more local disks, for example SATA disk drives, and control the reading and writing of data to and/or from these storage elements 408. Although the storage elements 408 above is described as a local disk, in general any other suitable computer-readable media such as a removable magnetic disk, optical storage media such as a CD or DVD, -ROM disk, solid state drives, flash memory cards, ... could be used. The system 400 described above can also run as a virtual machine above the physical hardware.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope of the claims. The present embodiments are therefore to be considered in all respects as illustrative and not restrictive, the scope of the invention being defined by the appended claims rather than by the foregoing description, and all changes which come within the meaning and range of equivalency of the claims are therefore intended to be embraced therein. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances.

## Claims

1. A method for preventing microbiological contamination (120) in a building (100) comprising a ventilation system (131-133), the method comprising the following steps:
- first detecting the contamination in a first ventilated sub-part (103) of the building by a VOC sensor (140) based on a first volume of air flowing through the ventilation system; and
- taking a first action in order to prevent further contamination; and
- performing a second measurement of the contamination based on a second larger volume of air from the sub-part supplied by the ventilation system upon the first detecting; and
- when the measurement is positive, taking a second action in order to prevent further contamination; and
- when the second measurement is negative, undoing the first action further **characterized in that** the performing a second measurement further comprises guiding the second larger volume of air through an air filter for a certain amount of time, extracting the air filter and performing a microbiological culture on air particles from the air filter.

2. The method according to claim 1 wherein the second action comprises removing a source that causes the contamination from the first ventilated sub-part.

3. The method according to claim 2 further comprising undoing the first action after the removing.

4. The method according to any one of the preceding claims wherein the taking the first action comprises adapting the ventilation system such that the sub-part is no longer ventilated thereby preventing contamination of other sub-parts of the building through the ventilation system.

5. The method according to claim 4 wherein the taking the first action further comprises adapting the ventilation such that other sub-parts that are in contact with the first ventilated sub-part are no longer ventilated.

6. The method according to any one of the preceding claims wherein the first ventilated sub-part is a patient room; and wherein taking the first action comprises preventing the patient from leaving the room.

7. The method according to any one of the preceding claims wherein the first ventilated sub-part comprises food products; and wherein taking the first action comprises marking the food products as possibly contaminated.

8. The method according to any one for the preceding claims wherein the taking a first action further comprises marking other sub-parts of the building through which air from the first ventilated sub-part flows as possibly contaminated.

## Patentansprüche

1. Verfahren zum Verhindern von mikrobiologischer Kontamination (120) in einem Gebäude (100), das ein Belüftungssystem (131-133) umfasst, wobei das Verfahren die folgenden Schritte umfasst:
- zuerst Erfassen der Kontamination in einem ersten belüfteten Unterteil (103) des Gebäudes durch einen VOC-Sensor (140) basierend auf einem ersten Luftvolumen, das durch das Belüftungssystem strömt; und
- Ergreifen einer ersten Maßnahme, um weitere Kontamination zu verhindern; und
- Durchführen einer zweiten Messung der Kontamination basierend auf einem zweiten größeren Luftvolumen aus dem Unterteil, das durch das Belüftungssystem bei dem ersten Erfassen zugeführt wird; und
- wenn die Messung positiv ist, Ergreifen einer zweiten Maßnahme, um weitere Kontamination zu verhindern; und
- wenn die zweite Messung negativ ist, Rückgängigmachen der ersten Maßnahme, ferner **dadurch gekennzeichnet, dass** das Durchführen einer zweiten Messung ferner das Leiten des zweiten größeren Luftvolumens durch einen Luftfilter für eine bestimmte Menge an Zeit, das Extrahieren des Luftfilters und das Durchführen einer mikrobiologischen Kultur an Luftpartikeln aus dem Luftfilter umfasst.

2. Verfahren nach Anspruch 1, wobei die zweite Maßnahme das Entfernen einer Quelle, die die Kontamination bewirkt, aus dem ersten belüfteten Unterteil umfasst.

3. Verfahren nach Anspruch 2, ferner umfassend das Rückgängigmachen der ersten Maßnahme nach dem Entfernen.

4. Verfahren nach einem der vorhergehenden Ansprüche, wobei das Ergreifen der ersten Maßnahme das Anpassen des Belüftungssystems umfasst, sodass das Unterteil nicht mehr belüftet wird, wodurch Kontamination anderer Unterteile des Gebäudes durch das Belüftungssystem verhindert wird.

5. Verfahren nach Anspruch 4, wobei das Ergreifen der ersten Maßnahme ferner das Anpassen der Belüftung umfasst, sodass andere Unterteile, die in Kontakt mit dem ersten belüfteten Unterteil sind, nicht mehr belüftet werden.

6. Verfahren nach einem der vorhergehenden Ansprüche, wobei der erste belüftete Unterteil ein Patientenzimmer ist; und wobei das Ergreifen der ersten Maßnahme das Verhindern des Verlassens des Zimmers durch den Patienten umfasst.

7. Verfahren nach einem der vorhergehenden Ansprüche, wobei der erste belüftete Unterteil Lebensmittelprodukte umfasst; und wobei das Ergreifen der ersten Maßnahme das Markieren der Lebensmittelprodukte als möglicherweise kontaminiert umfasst.

8. Verfahren nach einem der vorhergehenden Ansprüche, wobei das Ergreifen einer ersten Maßnahme ferner das Markieren anderer Unterteile des Gebäudes, durch die Luft von dem ersten belüfteten Unterteil strömt, als möglicherweise kontaminiert umfasst.

## Revendications

1. Procédé permettant de prévenir la contamination microbiologique (120) dans un bâtiment (100) comprenant un système de ventilation (131-133), le procédé comprenant les étapes suivantes :
- détection d'abord de la contamination dans une première sous-partie ventilée (103) du bâtiment par un capteur de COV (140) sur la base d'un premier volume d'air circulant à travers le système de ventilation ; et
- réalisation d'une première action afin d'empêcher une nouvelle contamination ; et
- réalisation d'une seconde mesure de la contamination sur la base d'un second plus grand volume d'air de la sous-partie fournie par le système de ventilation lors de la première détection ; et
- lorsque la mesure est positive, réalisation d'une seconde action afin de prévenir une nouvelle contamination ; et
- lorsque la seconde mesure est négative, l'annulation de la première action, **caractérisé en outre en ce que** la réalisation d'une seconde mesure comprend en outre le guidage du second plus grand volume d'air à travers un filtre à air pendant un certain temps, l'extraction du filtre à air et la réalisation d'une culture microbiologique sur des particules d'air provenant du filtre à air.

2. Procédé selon la revendication 1, ladite seconde action comprenant l'élimination d'une source qui entraîne la contamination à partir de la première sous-partie ventilée.

3. Procédé selon la revendication 2, comprenant en outre l'annulation de la première action après le retrait.

4. Procédé selon l'une quelconque des revendications précédentes, ladite réalisation de la première action comprenant l'adaptation du système de ventilation de sorte que la sous-partie ne soit plus ventilée, prévenant ainsi la contamination d'autres sous-parties du bâtiment par le système de ventilation.

5. Procédé selon la revendication 4, ladite réalisation de la première action comprenant en outre l'adaptation de la ventilation de sorte que les autres sous-parties qui sont en contact avec la première sous-partie ventilée ne soient plus ventilées.

6. Procédé selon l'une quelconque des revendications précédentes, ladite première sous-partie ventilée étant une chambre de patient ; et ladite réalisation de la première action comprenant la prévention de la sortie du patient de la chambre.

7. Procédé selon l'une quelconque des revendications précédentes, ladite première sous-partie ventilée comprenant des produits alimentaires ; et ladite réalisation de la première action comprenant le marquage des produits alimentaires comme potentiellement contaminés.

8. Procédé selon l'une quelconque des revendications précédentes, ladite réalisation d'une première action comprenant en outre le marquage d'autres sous-parties du bâtiment à travers lesquelles l'air provenant de la première sous-partie ventilée circule comme éventuellement contaminés.
